# EUROPEAN PATENT APPLICATION

(11) **EP 2 277 483 A1**
(43) Date of publication of application: **26.01.2011**
(21) Application number: 09746455.6
(22) Date of filing: 07.04.2009
(51) Int. Cl.: A61F 13/15, A61F 13/472, A61F 13/534, A61F 13/539

(54) **ABSORBENT ARTICLE**

(30) Priority: 15.05.2008 JP 2008128801
(71) Applicant: Unicharm Corporation, Ehime 799-0111 (JP)
(72) Inventor: SAGISAKA, Minako, Kanonji-shi Kagawa 769-1602 (JP); NISHITANI, Kazuya, Kanonji-shi Kagawa 769-1602 (JP)
(74) Representative: Knights, Rupert
(86) International application number: PCT/JP2009/057417
(87) International publication number: WO 2009/139248

(57) **Abstract**

To provide an absorbent article allowing no generation of twisting, realizing a wide contact area of the absorber with skin, and enabling the absorbing layer center part to always obtain a stable deformed state.

An absorbent article comprising at least a liquid-permeable sheet, a liquid-impermeable sheet, and an absorber sandwiched between the liquid-permeable sheet and the liquid-impermeable sheet, wherein:
the absorber consists of absorbing layer center part and an absorbing part formed in the periphery of the absorbing layer center part to have a lower basis weight than the absorbing layer center part;
on the skin-contact surface side of the absorbent article, compressed grooves are provided at least to exhibit symmetry with respect to the longitudinal centerline and surround the absorbing layer center part, the compressed grooves having a pair of right and left intermittent portions formed across the longitudinal centerline; and
on the skin-non-contact surface side of the absorber, a space part being nearly convex toward the skin-contact surface side and formed between the absorber and the liquid-impermeable sheet, is present in the side part of at least a part of a compressed groove out of the compressed grooves provided along widthwise right and left side edges.

## Description

### TECHNICAL FIELD

The present invention relates to an absorbent article. More specifically, the present invention relates to an absorbent article such as sanitary napkin and incontinence pad, in which a compressed groove is provided at a predetermined position of an absorber and a space part is provided in the side part of the compressed groove.

### BACKGROUND ART

As for the absorbent article aiming at preventing leakage of a body fluid, an absorbent article in which a three-dimensionally shaped protrusion is formed on the skin-contact surface and the periphery of the protrusion is continuously surrounded by a concave part is known (see, Japanese Unexamined Patent Publication (Kokai) No. 11-33054).

However, in such an absorbent article where the periphery of the protrusion is continuously surrounded by a concave groove, since the groove is formed by compressing a surface sheet and an adsorbing layer, the groove part has a higher density than the periphery and in turn the rigidity is increased, which makes it difficult to flexibly deform following the body shape.
In particular, in the rear end of the protrusion, which corresponds to the backward of the excretory part, the groove continues in the width direction of the absorber. Therefore, when a force is applied from the widthwise outside on fitting, the absorbent article can be hardly raised toward the wearer's groin.

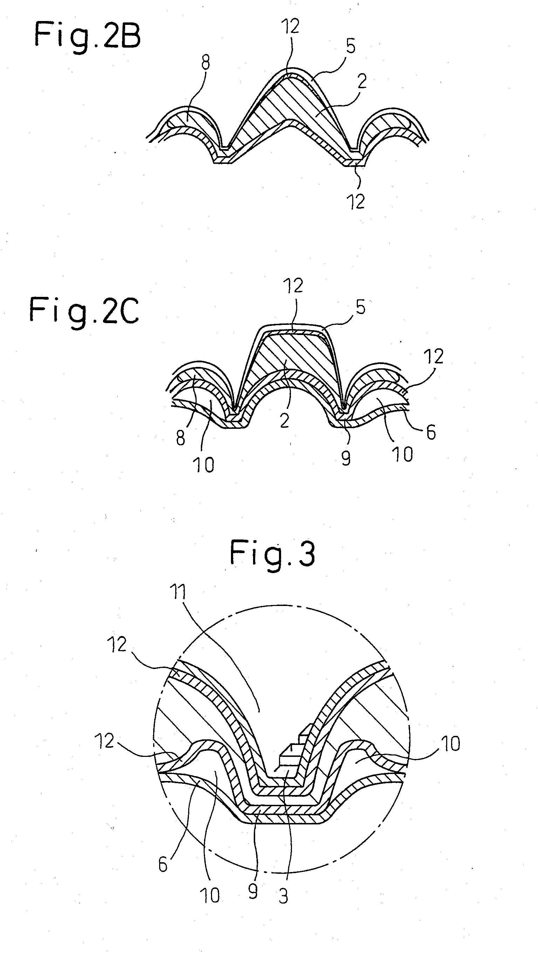

Even when raised, since the structure is not designed to specify the position of the wear's grion, the raised portion may not be at a position along the shape of the wearer' groin and this induces generation of twisting. Moreover, when the absorbent article is raised, the cross-sectional shape in the width direction becomes a chevron shape (see, Fig. 2B) and the area of the absorber contacting with the wearer's skin is extremely reduced.

Furthermore, since the groove part is disposed between the nearly center part in the thickness direction of the core part and the back surface of the core part, when a compressive force is applied in the width direction from the wearer's femoral region, the force transmitted to the core part through the groove diffuses from the skin-contact surface side in the thickness direction of the absorber to various directions such as skin-non-contact surface side. As a result, the core part may be bent in various directions, and it is impossible to always obtain a stable deformed state.

Also, for improving the leakage in the width direction and the instability of surface shape of the absorbing layer on fitting, an absorbent article where a groove part is separated into a plurality of parts has been reported (see, Japanese Unexamined Patent Publication (Kokai) No. 2007-195665). However, in this case, since the groove part is provided only in the absorbing layer on the skin-contact surface side, the absorber may be bent in various directions and it is impossible to always obtain a stable deformed state.

On the other hand, as for an absorbent article having a flexible axis not only in the absorbing layer on the skin-contact surface side but also in the absorbing layer on the skin-non-contact surface side, an absorbent article where a three-dimensional groove as a concave part is provided on the skin-non-contact surface side, by applying a grooving work from the skin-non-contact surface side toward the skin-contact surface side has been reported (see, Japanese Unexamined Patent Publication (Kokai) No. 10-99372). The three-dimensional groove is formed only on either one side, inside or outside, of the position on the skin-non-contact surface side corresponding to the three-dimensional groove on the skin-contact surface side. However, the absorbent article takes various fitting shapes when fitted to the groin. Therefore, depending on the fitting form, it is difficult for the absorbent article of Japanese Unexamined Patent Publication (Kokai) No. 10-99372 to always take a predetermined shape ensuring excellent fitting property.

### SUMMARY OF THE INVENTION

Accordingly, an object of the present invention is to provide an absorbent article allowing no generation of twisting, enabling the absorber to contact with the wearer's skin in a larger area, eliminating a fear of bending of the absorbing layer center part (core part) in various directions, and being capable of always realizing a stable deformed state.

Under these circumstances, the present inventors have made intensive studies, as a result, it has been found that when on the skin-contact surface side, compressed grooves symmetric with respect to the longitudinal centerline are provided to surround the absorbing layer center part, the compressed groove being separated at a specific position, and on the skin-non-contact surface side, a nearly convex space part is formed in the side part of at least a part of a compressed groove out of the compressed grooves along the widthwise right and left side edges to be located between the absorber and the liquid-impermeable sheet, a protruding part formed on or inside of the skin-non-contact surface in the thickness direction of the absorbent article readily enters the space part and the above-described object can be thereby attained. The present invention has been accomplished based on this finding.

That is, (1) the present invention provides an absorbent article comprising at least a liquid-permeable sheet, a liquid-impermeable sheet, and an absorber sandwiched between the liquid-permeable sheet and the liquid-impermeable sheet, wherein:
the absorber consists of an absorbing layer center part and an absorbing part formed in the periphery of the absorbing layer center part to have a lower basis weight compared to the absorbing layer center part;
on the skin-contact surface side of the absorbent article, compressed grooves are provided at least to exhibit symmetry with respect to the longitudinal centerline and surround the absorbing layer center part, the compressed grooves having a pair of right and left intermittent portions formed across the longitudinal centerline; and
on the skin-non-contact surface side of the absorber, a space part being nearly convex toward the skin-contact surface side and formed between the absorber and the liquid-impermeable sheet, is present in the side part of at least a part of a certain compressed groove out of the compressed grooves provided along widthwise right and left side edges.
   (2) The present invention provides the absorbent article as described in (1), wherein the intermittent portion is formed on the longitudinal front side.
   (3) The present invention provides the absorbent article as described in (2), wherein the intermittent portion is further formed on longitudinal back side.
   (4) The present invention provides the absorbent article as described in any one of (1) to (3), wherein the space part is formed on both side parts of at least a part of a compressed groove out of the compressed grooves provided along widthwise right and left side edge.
   (5) The present invention provides the absorbent article as described in any one of (1) to (4), wherein the space part is formed to extend to both ends of each compressed groove provided along widthwise right and left side edges.

According to the absorbent article of the present invention, a pair of right and left intermittent portions are provided across the longitudinal centerline, so that the absorbing layer center part can be raised toward the body while specifying the raising position in the absorbing layer center part and generation of twisting can be prevented. Also, the cross-sectional shape of the absorbing layer center part when raised becomes a trapezoidal shape, so that the absorbing area of the absorber can be made wide.

Furthermore, on the skin-non-contact surface side of the absorber, a space part is provided in the side part of the compressed groove, so that the protruding part provided on or inside of the skin-non-contact surface of the absorber can move to be pushed into the space part depending on the fitted state of the absorbent article and push up the absorbing layer center part from the skin-non-contact surface side and in turn the absorbent article can be always stably contacted to the wearer's excretory part to prevent leakage of the excreta.

### BRIEF DESCRIPTION OF THE DRAWINGS

Fig. 1 is a plan view of a representative absorbent article of the present invention.
Fig. 2A is an A-A' cross-sectional view of Fig. 1.
Fig. 2B is a view illustrating the cross-sectional shape when an absorbent article having formed therein a compressed groove without an intermittent portion is raised.
Fig. 2C is a view illustrating the A-A' cross-sectional shape when the absorbent article of Fig. 1 is raised.
Fig. 3 is an enlarged view of the circled region of Fig. 2A.
Fig. 4 is a plan view of an embodiment of the absorbent article of the present invention.
Fig. 5 is a plan view of another embodiment of the absorbent article of the present invention.
Fig. 6 is a plan view of another embodiment of the absorbent article of the present invention.
Fig. 7 is a plan view of another embodiment of the absorbent article of the present invention.
Fig. 8 is a plan view illustrating one embodiment of the space part 10 region of the absorbent article of Fig. 1 from the skin-non-contact surface side.

### EMBODIMENT FOR CARRYING OUT THE INVENTION

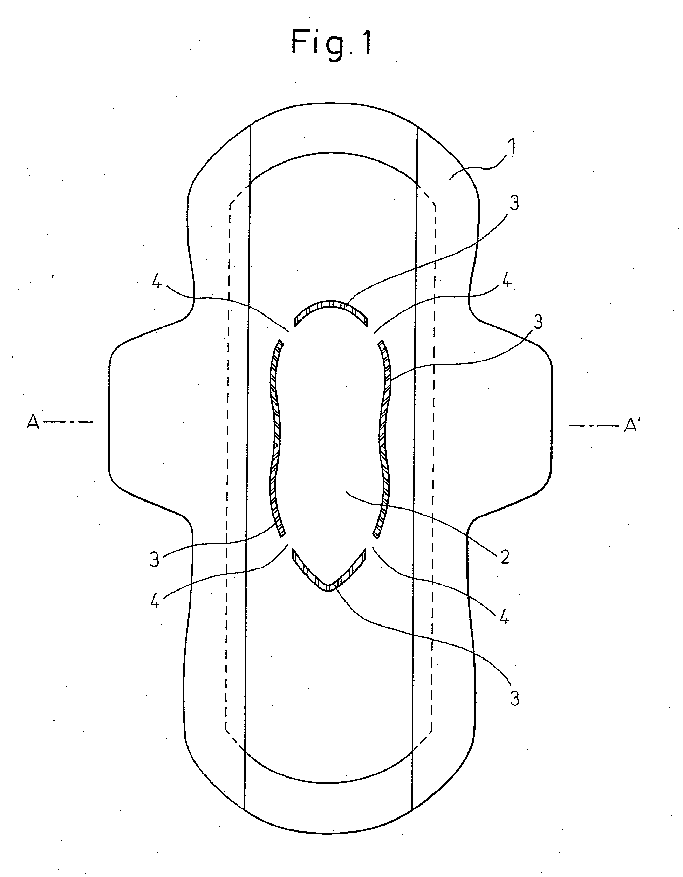

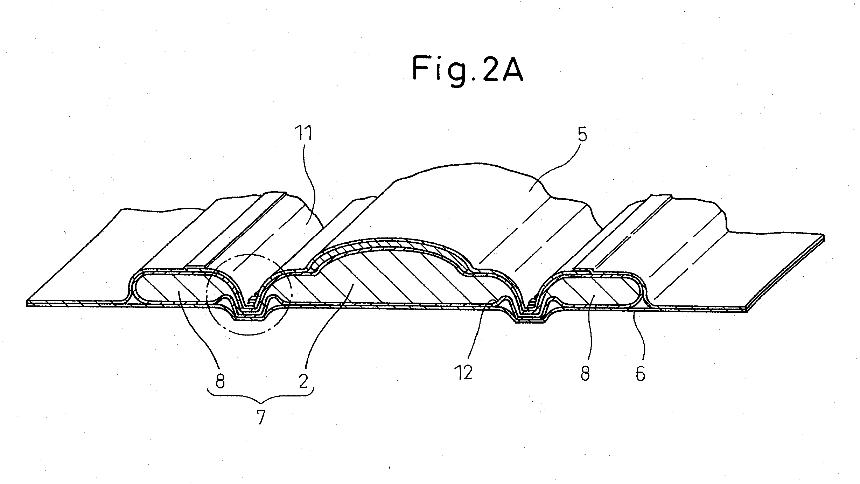

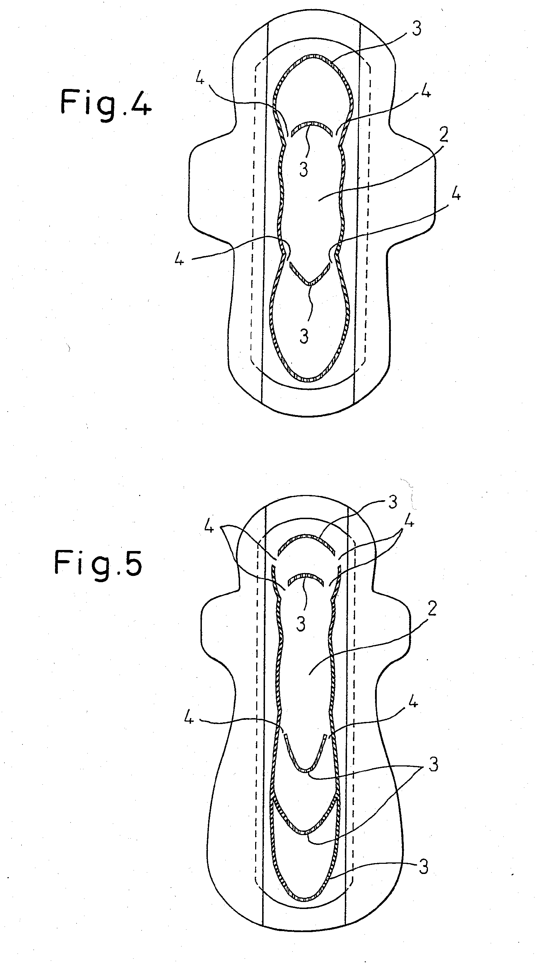

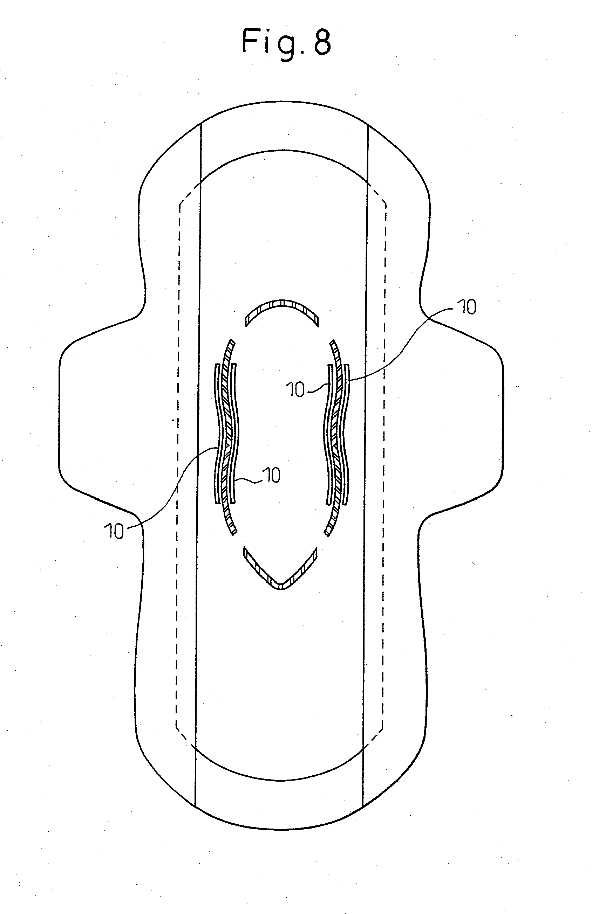

The present invention is described below by referring to the drawings, but the present invention is not limited to those illustrated in the drawings. Fig. 1 is a plan view of a representative absorbent article 1 of the present invention, and Fig. 2A is an A-A' cross-sectional view of Fig. 1. Fig. 2C is a view illustrating the A-A' cross-sectional shape when the absorbent article of Fig. 1 is raised. Fig. 2B is a view illustrating the cross-sectional shape when an absorbent article having formed therein a compressed groove without an intermittent portion is raised, for comparison with the absorbent article 1 of the present invention. Fig. 3 is an enlarged view of the circled region in Fig. 2A. Figs. 4 to 7 are plan views illustrating the embodiments of the absorbent article 1 of the present invention. Fig. 8 is a plan view illustrating one embodiment of the space part 10 region of the absorbent article of Fig. 1 from the skin-non-contact surface side.

Incidentally, in the absorbent articles of the present invention illustrated in these drawings, a pair of wing parts extending from both side edges in the longitudinal direction are provided, but the wing part may not be provided.

The absorbent article 1 of the present invention consists of at least a liquid-permeable sheet 5, a liquid-impermeable sheet 6, and an absorber 7. The absorber 7 is usually integrated with a mount 12, and the integrated absorber is sandwiched between the liquid-permeable sheet 5 and the liquid-impermeable sheet 6. The absorber 7 is composed of an absorbing layer center part 2 and an absorbing part 8 in the periphery thereof, and the peripheral absorbing part 8 is formed to have a lower basis weight than the absorbing layer center part 2. Although it may vary depending on the usage or the like of the absorber, the basis weight of the absorbing layer center part 2 is generally from 200 to 1,200 g/m², and the basis weight of the peripheral absorbing part 8 is from 100 to 400 g/m² The absorber 7 is not necessarily required to be composed of one absorbing layer, but may be divided into, for example, an absorbing part as the lower layer and a protrusion as the upper layer.

As illustrated in Fig. 1, in the absorbent article 1 of the present invention, when planarly viewed, compressed grooves 3 are formed at least to exhibit symmetry with respect to the longitudinal centerline and surround the absorbing layer center part 2. The liquid-permeable sheet 5 and the absorber 7 are joined together by the compressed grooves 3.

The shape of the compressed groove 3 may be sufficient if it is at least a shape surrounding the absorbing layer center part 2. For example, out of the compressed grooves surrounding the absorbing center part 2, the shape of each compressed groove formed along the widthwise right and left side edges may be any of, for example, a linear shape, a waved shape and a widthwise outwardly convex shape. Also, out of the compressed grooves surrounding the absorbing center part 2, the shape of the compressed groove on the longitudinal front and back sides may be any of, for example, a linear shape, a waved shape and a longitudinally outwardly convex shape. In addition, the shape of the compressed groove 3 may be, as illustrated in Figs. 4 to 7, a shape surrounding the absorbing layer center part 2 and further being longitudinally outwardly convex on the longitudinal front and back sides.

The shape of the groove, per se, in the compressed groove 3 is not particularly limited, and may be, for example, a concave part where the entire compressed groove has a constant depth. As illustrated in Fig. 3, the groove is preferably formed as an aggregate composed of a deepest part and a shallow part. When such a shape is taken, by virtue of the difference between the density in the deepest part and the density in the shallow part, effective indraft of excreta can be attained and not only the rigidity is prevented from becoming excessively high but also the texture is hardly impaired.

The shape of the absorbing layer center part 2 surrounded by compressed groves 3 includes, but is not limited to rectangular, elliptical, circular, droplet-like, quadrangular and triangular shapes. The dimension of the absorbing layer center part 2 surrounded by the compressed grooves 3 is preferably about 40 mm or more, more preferably from about 60 to about 120 mm, in the longitudinal direction, and preferably about 10 mm or more, more preferably from about 25 to about 50 mm, in the width direction. As for the positional relationship of the absorbing layer center part 2 surrounded by the compressed grooves 3, usually, the longitudinal front end of the absorbing layer center part 2 surrounded by the compressed grooves 3 is present at least 10 mm backward from the longitudinal front end of the absorber 7, and the longitudinal rear end of the absorbing layer center part 2 surrounded by the compressed grooves 3 is present at least 10 mm frontward from the longitudinal rear end of the absorber 7.

The compressed groove 3 has a pair of right and left intermittent portions 4 in which the absorber is not compressed and which are provided across the longitudinal centerline. The compressed groove along the widthwise left side edge and the compressed groove along the widthwise right side edge, which are separated by the intermittent portion 4, are referred to as a "widthwise left side edge compressed groove" and a "widthwise right side edge compressed groove", respectively, and these are sometimes collectively referred to as "widthwise right and left side edge compressed grooves". When a force is applied from the widthwise outside of the absorber on fitting, the absorbent article causes folding to the skin-non-contact surface side by using the widthwise right and left side edge compressed grooves, as the folding base point. On the other hand, in the portion widthwise inside of the widthwise right and left side edge compressed grooves and adjacent to the compressed groove, a pair of right and left intermittent potions are provided across the longitudinal centerline. Therefore, the intermittent portion is liable to be flexibly bent due to difference in rigidity between the compressed groove and the intermittent portion, and as a result, the absorbent article is easily raised to the skin-contact surface side. Furthermore, a compressed groove along the width direction is provided between the pair of right and left intermittent portions. Therefore, the absorbing layer center part surrounded by the compressed grooves is inevitably raised toward the wearer's body using the intermittent portions as the base point. Accordingly, the absorbing layer center part is raised in a predetermined shape and fits to the body (cleft of buttocks). The absorbent article can be thereby prevented from occurrence of twisting.

The intermittent portions 4 may be provided on either one side or both sides of the front side and the back side in the longitudinal direction, but are preferably provided at least on the longitudinal front side. Because the presence of intermittent portions on the longitudinal front side enables widening the surface area coming into contact with the shape of wearer's excretory part, and the absence of intermittent portions on longitudinal back side allows the absorbent article to rise and readily fit to the body. In Fig. 1, an embodiment where intermittent portions are provided on front and back both sides in the longitudinal direction is illustrated.

The length of the intermittent portion is usually from about 2 to about 20 mm, preferably from about 5 to about 10 mm. If the length of the intermittent portion is less than 2 mm, the intermittent portion can hardly function as the folding base point and this is not preferred. If it exceeds 20 mm, excreta when flows out from the absorbing layer center part cannot be stemmed and may disadvantageously leak out of the absorbent article.

The compressed groove 3 forms a protruding part 9 on the skin-non-contact surface side. The protruding part 9 is usually formed such that the protruding part 9 is at the same position as the skin-contact surface in the thickness direction or a position inside of the skin-contact surface in a state of the absorbent article being not fitted and protrudes from the skin-non-contact surface to the skin-non-conduct surface side in a fitted state. The protruding part 9 is preferably formed to protrude from the skin-non-contact surface to the skin-non-contact surface side already in a non-fitted state.

In the side part of at least a part of each of the widthwise left side edge compressed groove 3 and the widthwise right side edge compressed groove 3, a space part 10 nearly convex toward the skin-contact surface side is formed between the mount 12 and the liquid-impermeable sheet 6 such that the space part almost sandwiches the protruding part 9 when cross-sectionally viewed. As used herein, the term "side part" means a region in the vicinity of each compressed groove, including the side edge part of each of the compressed groove along the widthwise left side edge and the compressed groove along the widthwise right side edge. When a compressive force is applied from the wearer's femoral region, the protruding part 9 deforms and moves to the widthwise inner side, and the space part 10 is a portion into which the deformed and moved protruding part 9 is pushed. The thus-moved and pushed-in protruding part 9 pushes up the absorbing layer center part 2 from the skin-non-contact surface side of the absorbing layer center part 2, whereby the absorbing layer center part 2 deforms to protrude to the skin-contact surface side and closely contacts with the wearer's excretory part ("engagement effect").

In Fig. 2A and in Fig. 3 enlarging the circled region of Fig. 2A, an embodiment where the protruding part 9 protrudes from the skin-non-contact surface to the skin-non-contact surface side in a non-fitted state is illustrated.

In the case where the protruding part 9 protrudes from the skin-non-contact surface to the skin-non-contact surface side in a fitted state or where the protruding part 9 is formed to protrude from the skin-non-contact surface to the skin-non-contact surface side already in a non-fitted state, the protrusion degree of the protruding part 9 is to such an extent as allowing the bottom of the compressed groove 3 to protrude to the skin-non-contact surface side in a range of about 0.5 to about 10 mm from the skin-non-contact surface of the absorbing layer center part 2. That is, the protruding part protrudes such that the difference of height in the thickness direction between the bottom of the compressed groove 3 and the skin-non-contact surface of the absorbing layer center part 2 becomes from about 0.5 to about 10 mm. This difference is preferably about 1 mm. When this difference is from about 0.5 to about 10 mm, the protruding part 9 can easily enter the space part 10.

The space part 10 is formed to locate its top closer to the skin-contact surface side by about 0.5 to about 10 mm than the skin-non-contact surface of the absorbing layer center part 2. That is, the space part is formed such that the difference of height in the thickness direction of the absorber 7 between the bottom of the compressed groove 3 and the top of the space part 10 becomes from about 0.5 to about 10 mm. This difference is preferably about 2 mm. When this difference is from about 0.5 to about 10 mm, the protruding part 9 can easily enter the space part 10.

For example, as illustrated in Fig. 8, the space part 10 is sufficient if it is provided in at least a part of each of the widthwise right and left side edge compressed grooves 3, and the region thereof is not particularly limited. However, the space part 10 needs to be formed at least in a portion subject to a lateral pressure from legs. Therefore, in the case where the absorbent article is a sanitary napkin, the space part 10 is preferably formed at least in a region that comes into contact with ostium vaginae. In this case, the length in the longitudinal direction of the space part 10 is at least 15 mm or more. If the length is less than 15 mm, the "engagement effect" can be hardly obtained. In the absorbent article of the present invention, the space part is preferably formed to extend to both ends of each of the widthwise right and left side edge compressed grooves 3 (not shown), because the flow of excreta can be led to the absorbing layer center part. The space part 10 is more preferably formed to, as described later in the first to fourth embodiments, extend in the longitudinal direction to a position of the compressed groove outside of the intermittent portion present on the longitudinal front side or front and back sides, including the widthwise right and left side edge compressed grooves (not shown). Incidentally, as used herein, the term "end" of the compressed groove means a terminal portion of each of the widthwise right and left side edge compressed grooves.

The space part 10 is provided in at least a part of each of the widthwise right and left side edge compressed grooves 3 out of the compressed grooves 3 surrounding the absorbing layer center part 2 but need not be always provided in both side parts of the compressed groove.

For example, the space part 10 may be formed in only one side part of each compressed groove 3, that is, may be formed in one side part of the widthwise left side edge compressed groove 3 and one side part of the widthwise right side edge compressed groove 3 to make a pair and be located between the widthwise left side edge compressed groove 3 and the widthwise right side edge compressed groove 3. By such a construction, when a force is laterally applied on fitting, the absorbing layer center part 2 is lifted to the wearer side using the space part 10 as the base point and on the other hand, the protruding part 9 deforms and moves to the widthwise inner side to push up and raise the absorbing center part 2, allowing the absorbing layer center part 2 to readily fit to the body. In this case, the pair of space parts 10 are preferably disposed at a position closer to the above-described pair of compressed grooves 3 than to the longitudinal centerline, whereby a stable deformed form can be ensured.

Preferably, the space part 10 is provided in both side parts of each compressed groove 3. Because when a lateral pressure is applied, the protruding part moves nearly in parallel to the absorbing layer center part side to allow a force to be easily transmitted in a constant direction from the compressed groove to the absorbing layer center part and in turn the cross-sectional shape of the absorbing layer center part when raised can be maintained as a bilaterally symmetric trapezoidal shape (see, Fig. 2C), instead of the chevron shape of Fig. 2B. In such a absorbing layer center part of trapezoidal shape, the absorber can provide a wide absorbing area. Furthermore, by virtue of providing the space part also in the side part on the non-absorbing layer center part side of the compressed groove, the space part can unfailingly push up the absorber portion outside of the compressed groove, and therefore the excreta can be stemmed by the compressed groove without fail. In this way, formation of a space part in both side parts enables ensuring a more stable deformed form compared to the formation of a space part in one side part and enhancing the close contact to the skin. In Fig. 3, an embodiment where a space part 10 is provided in both side parts of a protruding part 9 to sandwich the protruding part is illustrated.

The compressed groove 3 has a pair of wall parts 11 formed by the engagement of an embossing apparatus described later (see, Figs. 2A and 3). For example, when the liquid-permeable sheet 5 and the absorber 7 are compressed to the thickness direction of the absorbent article 1, a strong tensile stress is generated in nearly the center part in the thickness direction of the absorbent article 1 and the absorber 7 is stretched by the tensile stress, whereby the wall part 11 is formed. The density of the wall part 11 formed resulting from the absorber 7 being stretched is low in nearly the center part thereof. This low-density portion can suppress diffusion of the excreta running from the high-density portion when the absorbing layer center part 2 absorbs the excreta.

Figs. 4 to 7 illustrate embodiments (first, second, third and fourth embodiments, respectively) of the absorbent article 1 of the present invention. In the first to fourth embodiments, in addition to the compressed grooves 3 illustrated in Fig. 1, a compressed groove 3 in a longitudinally outwardly convex shape is provided on the longitudinal front and back sides of the absorbent article 1. Also in the first to fourth embodiments, all compressed grooves 3 are provided to exhibit bilateral symmetry with respect to the longitudinal centerline as the base point, similarly to the embodiment illustrated in Fig. 1.

In the first embodiment, as illustrated in Fig. 4, a compressed groove is further connected to the end of the widthwise left side edge compressed groove 3, while this compressed groove connected draws a longitudinally outwardly convex shape, the compressed groove is connected to the end of the widthwise right side edge compressed groove. Such a convex compressed groove is formed on front and back both sides in the longitudinal direction. In this embodiment, the compressed groove is provided in a closed chain form in the circumferential periphery of the entire absorber.

In this embodiment, the absorbing layer center part is raised by using the intermittent portions as the base points, and symmetric deformation with respect to the base point assigned to the longitudinal centerline in the absorbing layer center part is transmitted as it is to the longitudinal front and back sides, whereby generation of twisting can be prevented. The compressed groove where the liquid-permeable sheet and the absorbing layer center part are compressed, has a higher density than the peripheral absorbing part and has a force of drawing excreta. Therefore, even if excreta is leaked from the absorbing layer center part, the excreta is led toward the compressed groove on the longitudinal front or back side, whereby leakage of excreta to the outside of the absorbent article can be prevented by efficiently utilizing the entire surface of the absorber.

In the second embodiment, as illustrated in Fig. 5, the compressed groove is provided in nearly the same closed chain form as in the first embodiment. However, in the second embodiment, the compressed groove 3 surrounding the absorbing layer center part 2 is formed at a position slightly closer to the longitudinal front side as compared with the first embodiment. Also, on the longitudinal back side, a compressed groove in a longitudinally outwardly convex shape is further formed between the longitudinal backside compressed groove out of the compressed grooves 3 surrounding the absorbing layer center part 2 and the compressed groove formed in a longitudinally outwardly convex shape in the first embodiment.

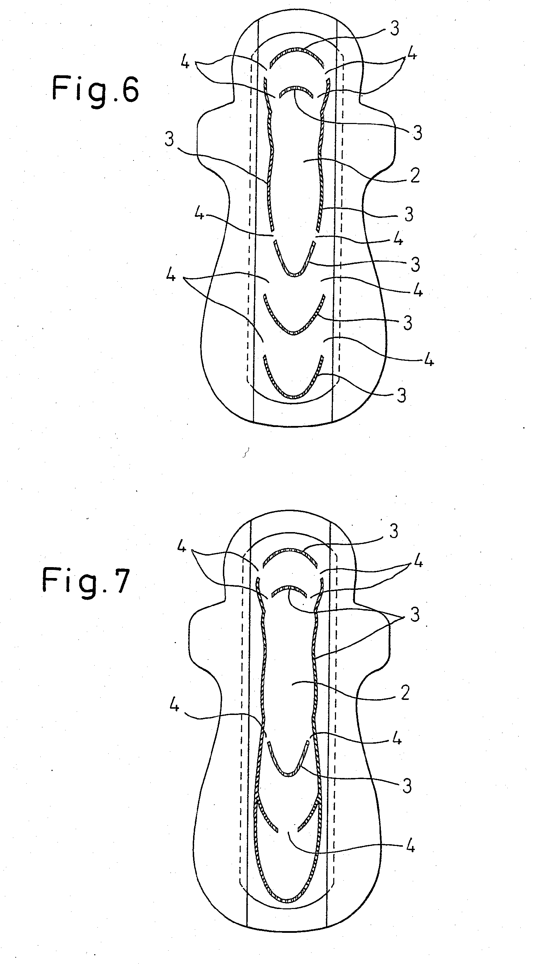

In the third embodiment, as illustrated in Fig. 6, the compressed groove in a closed chain form in the second embodiment is separated at the position having the longitudinal rear end of each of the widthwise right and left side edge compressed grooves 3. More specifically, on the longitudinal back side of the longitudinal backside compressed groove 3 surrounding the absorbing layer center part 2, two compressed grooves formed in a longitudinally outwardly convex shape each has an intermittent portion 4 in the widthwise left side edge and the widthwise right side edge such that the intermittent portions exhibit bilateral symmetry with respect to the longitudinal centerline.

In the fourth embodiment, as illustrated in Fig. 7, a compressed groove in a longitudinally outwardly convex shape formed between the longitudinal backside compressed groove out of the compressed grooves 3 surrounding the absorbing layer center part 2 and the compressed groove formed in a longitudinally outwardly convex shape in the first embodiment has an intermittent portion 4 on the longitudinal centerline.

In the second to fourth embodiments, one or more compressed grooves are further provided on the longitudinal back side of the widthwise right and left side edge compressed grooves. Therefore, even if excreta is leaked from the absorbing layer center part, the excreta can be led to the compressed groove on the longitudinal back side.

Also, in the first, second and fourth embodiments, a compressed groove is formed widthwise outside of the front and back intermittent portions in the absorber region surrounding the absorbing layer center part 2 and therefore, when a force is applied from the widthwise outside, the absorber can be easily raised due to rigidity difference between the compressed groove and the intermittent portion. Furthermore, in the case where the space part is formed to extend to the position of the compressed groove outside of front and back intermittent portions in the longitudinal direction, the protruding part deforms and moves to the widthwise inner side to push up and raise the absorbing layer center part and therefore, the absorbing layer center part is readily fit to the body. Incidentally, in the third embodiment, when the space part is formed to extend to the position of the compressed groove outside of the front intermittent portion in the longitudinal direction, the same effect as above can be obtained.

In these first to fourth embodiments, the dimension of absorbing layer center part, the shape of groove, per se, of the compressed groove, the length of intermittent portion, the length of space part, the positional relationship between the top of space part and the bottom of compressed groove, and the difference of height in the thickness direction between the compressed groove and the skin-non-contact surface of absorbing layer center part are the same as those in the embodiment of a representative absorbent article of the present invention illustrated in Fig. 1.

The liquid-permeable sheet is entirely or partially liquid-permeable, and the liquid permeation area is formed of, for example, a resin film having formed therein a large number of liquid permeation cells, a net-like sheet having a large number of meshes, a liquid-permeable nonwoven fabric or a woven fabric. As for the resin film or net-like sheet, those formed from polypropylene, polyethylene, polyethylene terephthalate or the like may be used. As to the nonwoven fabric, for example, a spunlaced nonwoven fiber formed from a cellulose fiber such as rayon, a synthetic resin fiber or the like, and an air-through nonwoven fiber formed from the synthetic resin fiber above may be used. Also, in combination with forming a large number of liquid permeation cells, a silicone-containing or fluorine-containing water-repellent oily agent may be coated on the liquid-permeable sheet to deter attachment of a body fluid to the outer surface.

The diameter of the fiber above is preferably from 1.1 to 6.6 dtex, and the basis weight of the liquid-permeable sheet is preferably from 15 to 120 g/m².

The absorber constituting the absorbent article of the present invention is composed of, for example, pulp, chemical pulp, rayon, acetate, natural cotton, polymer absorber, fibrous polymer absorber, synthetic fiber, foam or a mixture thereof, and its material that hardly loses shape and causes less chemical irritation is preferred. The material above is stacked, and the stack is covered by winding a sheet material (covering material) therearound or sandwiching the stack between top and bottom two sheets and then pressed to form an absorber. Examples of the covering material include a cellulose-based fiber such as pulp, chemical pulp, rayon, acetate and natural cotton, and a thermoplastic resin such as polyethylene, polypropylene and polyethylene terephthalate.

In the case where the absorber has a protrusion, the protrusion may be formed by disposing another absorber on an absorber composed of one or more layers or causing the basis weight to be partially higher than the periphery within a one-layer absorber.

The liquid-impermeable sheet is sparingly permeable to a liquid and does not pass a body fluid. Examples thereof include polyethylene, polypropylene, polyethylene terephthalate, polyvinyl alcohol, polylactic acid, polybutyl succinate, nonwoven fabric, paper and a laminated material thereof, each having a thickness of 15 to 60 µm. A breathable film obtained by filling an inorganic filler and applying stretching may also be used. Specific examples thereof include a film prepared using a low-density polyethylene resin as the main component and adjusted to have a basis weight of 15 to 30 g/m².

The absorbent article of the present invention can be used as a sanitary napkin, an incontinence pad or the like.

The production method of the absorbent article of the present invention comprises at least an embossing step of overlapping a liquid-permeable sheet 5 and an absorber 7 and of ompressing these in the thickness direction to surround the absorbing layer center part 2. Thereby, a compressed groove 3 protruding from the skin-non-contact surface to the skin-non-contact surface side is formed. More specifically, in the embossing step, using an embossing apparatus comprising an upper roll part having on the surface thereof a convex part formed in a predetermined pattern and a lower roll part having formed on the surface thereof a concave part with which the convex part can engage the absorbent material obtained by overlapping a liquid-permeable sheet 5 and an absorber 7 is disposed between the upper and lower roll parts arranged to engage with, such that the liquid-permeable sheet 5 is put into contact with the upper roll part, and the absorbent material is then compressed. If desired, a non-woven fabric or an air-laid pulp may be inserted as a second sheet between the liquid-permeable sheet 5 and the absorber 7.

An adhesive layer, for example, a layer formed of a hot-melt resin or a hot-melt adhesive, may be provided between the liquid-permeable sheet 5 and the absorber 7 or between the second sheet and the absorber 7. In the case of providing a layer formed of a hot-melt resin or a hot-melt adhesive, the embossing work is preferably heat embossing. Simultaneously with forming a compressed groove by this embossing, the liquid-permeable sheet 5 and the absorber 7 may be integrated.

In the case of providing a wing part, for example, a wing member may be joined by inserting it between the liquid-permeable sheet 5 and the liquid-impermeable sheet 6, or the liquid-permeable sheet 5 may be extended to the width direction without disposing a wing member. Examples of the wing member include a nonwoven fabric and a film.

Finally, a liquid-impermeable sheet 6 is fed to the skin-non-contact surface side of the absorber 7, and the liquid-permeable sheet 5 and the liquid-impermeable sheet 6 are then joined together.

In the embodiment above, the production method of a sanitary napkin is described as an example, but the production method of the present invention can be applied to production of other absorbent articles such as incontinence pad.

## Claims

1. An absorbent article comprising at least a liquid-permeable sheet, a liquid-impermeable sheet, and an absorber sandwiched between said liquid-permeable sheet and said liquid-impermeable sheet, wherein:
said absorber consists of an absorbing layer center part and an absorbing part formed in the periphery of said absorbing layer center part to have a lower basis weight compared to said absorbing layer center part;
on the skin-contact surface side of said absorbent article, compressed grooves are provided at least to exhibit symmetry with respect to the longitudinal centerline and surround said absorbing layer center part, said compressed grooves having a pair of right and left intermittent portions formed across the longitudinal centerline; and
on the skin-non-contact surface side of said absorber, a space part being nearly convex toward the skin-contact surface side and formed between the absorber and the liquid-impermeable sheet, is present in the side part of at least a part of a compressed groove out of the compressed grooves provided along widthwise right and left side edges.

2. The absorbent article according to claim 1, wherein said intermittent portion is formed on the longitudinal front side.

3. The absorbent article according to claim 2, wherein said intermittent portion is further formed on the longitudinal back side.

4. The absorbent article according to any one of claims 1 to 3, wherein said space part is formed on both side parts of at least a certain compressed groove out of the compressed grooves provided along widthwise right and left side edges.

5. The absorbent article according to any one of claims 1 to 4, wherein said space part is formed to extend to both ends of each compressed groove provided along widthwise right and left side edges.
